# EUROPEAN PATENT SPECIFICATION

(11) **EP 3 965 838 B1**
(45) Date of publication and mention of the grant of the patent: **23.07.2025**
(21) Application number: 20838789.4
(22) Date of filing: 11.12.2020
(51) Int. Cl.: A61L 27/04, A61L 27/18, A61L 27/36, A61L 27/44

(54) **HYBRID ANNULOPLASTY RING**
HYBRIDER ANULOPLASTIKRING
ANNEAU D'ANNULOPLASTIE HYBRIDE

(30) Priority: 12.12.2019 US 201962947454 P
(43) Date of publication of application: 16.03.2022
(73) Proprietor: Edwards Lifesciences Corporation, Irvine, CA 92614 (US)
(72) Inventor: MIRAKI, Manouchehr A., Irvine, CA 92614 (US); HAN, Jingjia, Irvine, CA 92614 (US); TIAN, Bin, Irvine, CA 92604 (US); DA LA FUENTE, Angela B., Irvine, CA 92614 (US); GUI, Liqiong, Irvine, CA 92614 (US); SHANG, Hao, Irvine, CA 92614 (US); HOANG, Lien Houng Thi, Irvine, CA 92614 (US); DO, Vicky Hong, Irvine, CA 92614 (US)
(74) Representative: Smith, Jeremy Robert
(86) International application number: PCT/US2020/064443
(87) International publication number: WO 2021/119391

(56) References cited:
- WO-A2-2009/085199
- US-A1- 2002 173 343
- US-A1- 2006 217 804
- US-A1- 2011 221 096

## Description

### CROSS REFERENCE TO RELATED APPLICATION

This application claims the benefit of U.S. Patent Application No. 62/947454, filed December 12, 2019.

### TECHNICAL FIELD

This disclosure relates generally to apparatuses for heart valve repair and, more particularly, to annuloplasty rings comprising bioprosthetic tissue.

### BACKGROUND

The human heart generally includes four valves. Of these valves, the mitral valve is located in the left atrioventricular opening and the tricuspid valve is located in the right atrioventricular opening. Both of these valves are intended to prevent regurgitation of blood from the ventricle into the atrium when the ventricle contracts. In preventing blood regurgitation, both valves must be able to withstand considerable back pressure as the ventricle contracts. The valve cusps are anchored to the muscular wall of the heart by delicate but strong fibrous cords in order to support the cusps during ventricular contraction. Furthermore, the geometry of the heart valves ensure that the cusps overlay each other to assist in controlling the regurgitation of the blood during ventricular contraction.

Diseases and certain natural defects to heart valves can impair the functioning of the cusps in preventing regurgitation. For example, certain diseases cause the dilation of the heart valve annulus. Dilation may also cause deformation of the valve geometry or shape displacing one or more of the valve cusps from the center of the valve. Other diseases or natural heart valve defects result in deformation of the valve annulus with little or no dilation.

Dilation and/or deformation result in the displacement of the cusps away from the center of the valve. This results in an ineffective closure of the valve during ventricular contraction, which results in the regurgitation or leakage of blood during ventricle contraction. For example, diseases such as rheumatic fever or bacterial inflammations of the heart tissue can cause distortion or dilation of the valvular annulus. Other diseases or malformations result in the distortion of the cusps, which will also lead to ineffective closure of the valve.

Various surgical procedures have been developed to correct the deformation of the valve annulus and retain the intact natural heart. These surgical techniques involve repairing the shape of the dilated or elongated valve. Such techniques, generally known as annuloplasty, require surgically restricting the valve annulus to minimize dilation. Typically, a prosthesis is sutured about the base of the valve leaflets to reshape the valve annulus and restrict the movement of the valve annulus during the opening and closing of the valve.

A suitable prosthesis should allow the surgeon to properly reconstruct the heart valve annulus and minimize dilation, while allowing natural movement of the valve annulus during the opening and closing of the valve. The ability of the prosthesis to allow for a natural opening and closing of the valve is particularly important since such prostheses are not normally removed from the heart valve, even if the valve annulus heals to a normal geometry.

Many different types of prostheses have been developed for use in
annuloplasty surgery. In general prostheses are annular or partially annular shaped members which fit about the base of the valve annulus. Initially the prostheses were designed as rigid frame members, to correct the dilation and reshape the valve annulus to the natural state. These annular prostheses were formed from a metallic or other rigid material, which flexes little, if at all, during the normal opening and closing of the valve.

Current annuloplasty rings are typically comprised of a silicone and metal base that is wrapped with a sewing ring made of cloth. Repair of the valve annulus using current annuloplasty rings can, however, fail due to ring dehiscence at the implanting suture line and/or as a result of fibrous tissue overgrowth or pannus that can be triggered by the host response to the annuloplasty ring.

What is therefore desired are devices and methods for repairing a valve annulus which reduce the likelihood of dehiscence and which promote sufficient host tissue ingrowth to stabilize device once implanted in the host.

WO 2009/085199 describes a treatment for bioprosthetic tissue used in implants or for assembled bioprosthetic heart valves to reduce *in vivo* calcification. The method includes applying a calcification mitigant such as a capping agent or an antioxidant to the tissue to specifically inhibit oxidation in tissue. The tissue may comprise glutaraldehyde-fixed bovine pericardial tissue. US2002/01373843 A1 discloses a heart valve prosthesis comprising a harvested tissue heart valve with integral leaflets, the heart valve having an annulus at one end of the valve, and a belt secured around at least a substantial portion of an outer circumference of the annulus of the harvested tissue heart valve.

### SUMMARY

The scope of the invention is defined by the claims. Any "embodiment", "example", or "aspect" which is disclosed in the description but is not covered by the claims should be considered as presented for illustrative purposes only.

The present disclosure includes an annuloplasty ring prosthesis comprising a frame and a cover surrounding an outer surface of the frame. The cover comprises a bioprosthetic tissue.

In the presently claimed invention, the cover comprises a sheet of bioprosthetic tissue, the sheet having a first edge and a second edge. The sheet covers the outer surface of the frame, and the first edge and the second edge are joined together to form a seam. In an example, the cover can comprise a plurality of sheets that abut each other at abutment seams. In the presently claimed invention, the sheet is dimensioned to permit the first edge and the second edge of the sheet to fold or roll upon each other to form a lip. The lip protrudes away from the outer surface of the frame.

In the presently claimed invention, the bioprosthetic tissue is fixed and non-regenerative. In an example, the fixed, non-regenerative bioprosthetic tissue can be selected from the group consisting of pericardium, blood vessels, skin, dura mater, small intestinal submucosa, ligaments, tendons, muscle, ureter, urinary bladder, liver, and heart. In a further example, the fixed, non-regenerative bioprosthetic tissue can be a pericardium. In an additional example, the fixed, non-regenerative bioprosthetic tissue can be fixed with an aldehyde. In yet another example, the aldehyde can be a glutaraldehyde.

In one example, the free aldehyde groups in the fixed, non-regenerative bioprosthetic tissue can be subjected to a capping treatment comprising a capping agent. In one example, the capping agent can comprise an amine. In another example, the capping treatment can further comprise a reducing agent. In an additional example, the reducing agent can be a borohydride. In yet another example, the fixed, non-regenerative bioprosthetic tissue can be plasticized. In one example, the fixed, non-regenerative bioprosthetic tissue can be plasticized with a polyol. In another example, the polyol can be a glycerol.

In one example, not covered by the claims, the bioprosthetic tissue can be regenerative. In another example, not covered by the claims, the regenerative bioprosthetic tissue can be a decellularized biological tissue. In a further example, not covered by the claims, the decellularized tissue can be selected from the group consisting of: pericardium, blood vessels, skin, dura mater, small intestinal submucosa, ligaments, tendons, muscle, ureter, urinary bladder, liver, and heart. In an additional example, not covered by the claims, the regenerative bioprosthetic tissue can be an artificial scaffold. In yet another example, not covered by the claims, the artificial scaffold can be a biodegradable polymer scaffold. In one example, not covered by the claims, the biodegradable polymer scaffold can comprise a polyglycolic acid. In another example, not covered by the claims, the artificial scaffold can further comprise an extracellular matrix protein. In another example, not covered by the claims, the extracellular matrix protein can be one or more proteins selected from the group consisting of: hydroxyproline, vitronectin, fibronectin, collagen I, collagen III, collagen IV, collagen VI, collagen XI, collagen XII, fibrillin I, tenascin, decorin, byglycan, versican, asporin, agrin, and combinations thereof.

In one example, the frame can comprise one or both of a non-degradable polymer and a non-degradable metal or metal alloy. In another example, the frame can comprise a non-degradable metal or metal alloy selected from the group consisting of: stainless steel, a nickel-based alloy, a cobalt-chromium alloy, a nickel-cobalt-chromium alloy, nitinol, and combinations thereof.

In one example, the frame can be bioabsorbable. In another example, the bioabsorbable frame can comprise a metal or a metal alloy. In another example, the metal or the metal alloy can comprise one or a combination selected from the group consisting of magnesium, aluminum, iron, and zinc. In a further example, the metal or the metal alloy can have an ultimate tensile strength of about 30 MPa to about 400 MPa. In an additional example, the metal or the metal alloy can have an elongation of about 0.3 percent to about 170 percent. In yet another example, the bioabsorbable frame can be a bioabsorbable material. In one example, the bioabsorbable material can be one or a combination of polymers selected from the group consisting of: poly(L-lactide), poly(D-lactide), polyglycolide, poly(L-lactide-co-glycolide), polyhydroxyalkonate, polysaccharides, polyesters, polyhydroxyalkanoates, polyalkelene esters, polyamides, polycaprolactone, polylactide-co-polycaprolactone, polyvinyl esters, polyamide esters, polyvinyl alcohols, modified derivatives of caprolactone polymers, polytrimethylene carbonate, polyacrylates, polyethylene glycol, terminal dials, poly(L-lactide-co-trimethylene carbonate), polyhydroxybutyrate, polyhydroxyvalerate, poly-orthoesters, poly-anhydrides, polyiminocarbonate, and copolymers. In another example, the bioabsorbable frame can be reinforced with a reinforcing composition. In a further example, the reinforcing composition can comprise magnesium or a magnesium alloy.

The present disclosure further includes a sewing ring for a prosthetic heart valve, not falling within the scope of the claimed subject-matter. The sewing ring comprises a suture-permeable annular member and a cover surrounding an outer surface of the suture-permeable annular member. The cover comprises a bioprosthetic tissue.

In one example, not falling within the scope of the claimed subject-matter, the cover can comprise a sheet of bioprosthetic tissue, the sheet having a first edge and a second edge. The sheet can cover the outer surface of the suture-permeable annular member, and the first edge and the second edge can be joined together to form a seam. In another example, the cover can comprise a plurality of sheets that abut each other at abutment seams. In a further example, the sheet can be dimensioned to permit the first edge and the second edge of the sheet to fold or roll upon each other to form a lip. In an additional example, the lip can protrude away from the outer surface of the suture-permeable annular member.

In one example, not falling within the scope of the claimed subject-matter, the suture-permeable annular member can be molded from a suture-permeable, biocompatible polymer. In another example, the biocompatible polymer can be silicone. In a further example, the annular member can comprise a molded polymer. In an additional example, the molded polymer can be selected from the group consisting of: silicone, polyurethane, and combinations thereof.

In one example, not falling within the scope of the claimed subject-matter, the bioprosthetic tissue can be fixed and non-regenerative. In another example, the fixed, non-regenerative bioprosthetic tissue can be selected from the group consisting of pericardium, blood vessels, skin, dura mater, small intestinal submucosa, ligaments, tendons, muscle, ureter, urinary bladder, liver, and heart. In a further example, the fixed, non-regenerative bioprosthetic tissue can be a pericardium. In an additional example, the fixed, non-regenerative bioprosthetic tissue can be fixed with an aldehyde. In yet another example, the aldehyde can be a glutaraldehyde.

In one example, not falling within the scope of the claimed subject-matter, the free aldehyde groups in the fixed, non-regenerative bioprosthetic tissue can be subjected to a capping treatment comprising a capping agent. In one example, the capping agent can comprise an amine. In another example, the capping treatment can further comprise a reducing agent. In an additional example, the reducing agent can be a borohydride. In yet another example, the fixed, non-regenerative bioprosthetic tissue can be plasticized. In one example, the fixed, non-regenerative bioprosthetic tissue can be plasticized with a polyol. In another example, the polyol can be a glycerol.

In one example, not falling within the scope of the claimed subject-matter, the bioprosthetic tissue can be regenerative. In another example, not falling within the scope of the claimed subject-matter, the regenerative bioprosthetic tissue can be a decellularized biological tissue. In a further example, the decellularized tissue can be selected from the group consisting of: pericardium, blood vessels, skin, dura mater, small intestinal submucosa, ligaments, tendons, muscle, ureter, urinary bladder, liver, and heart. In an additional example, not falling within the scope of the claimed subject-matter, the regenerative bioprosthetic tissue can be an artificial scaffold. In yet another example, not falling within the scope of the claimed subject-matter, the artificial scaffold can be a biodegradable polymer scaffold. In one example, not falling within the scope of the claimed subject-matter, the biodegradable polymer scaffold can comprise a polyglycolic acid. In another example, the artificial scaffold can further comprise an extracellular matrix protein. In another example, not falling within the scope of the claimed subject-matter, the extracellular matrix protein can be one or more proteins selected from the group consisting of: hydroxyproline, vitronectin, fibronectin, collagen I, collagen III, collagen IV, collagen VI, collagen XI, collagen XII, fibrillin I, tenascin, decorin, byglycan, versican, asporin, agrin, and combinations thereof.

The present disclosure further includes a ring prosthesis, not falling within the scope of the claimed subject-matter. The ring prosthesis can comprise an elongated rod member that can be formed into a substantially ring shape. The elongated rod member can have a rod body, first and second ends and a free edge between the first and second ends. The free edge of the rod member can be secured to the rod body. The elongated rod member can be formed from a substantially flat bioprosthetic tissue having a length, a width, a first surface and a second surface opposing the first surface.

In one example, the second surface of the bioprosthetic tissue can have a texture that is smoother than the first surface of the bioprosthetic tissue. In another example, the first surface of the bioprosthetic tissue can have a texture that is rougher than the second surface of the bioprosthetic tissue. In one example, the first surface of the bioprosthetic tissue can form an external surface of the rod body. In a further example, the second surface of the bioprosthetic tissue can form an external surface of the rod body.

In an additional example, the free edge can be secured to the rod body with sutures. In accordance with this example, the ring prosthesis can consist of the substantially flat bioprosthetic tissue and sutures. In a further example, the free edge can be secured to the rod body with an adhesive. In accordance with this further example, the ring prosthesis can consist of the substantially flat bioprosthetic tissue and the adhesive.

In a further example, the ring prosthesis may not have a frame or a support. In another example, the ring prosthesis can consist essentially of the bioprosthetic tissue. In a further example, the rod member consists essentially of the bioprosthetic tissue.

In yet another example, the first and second ends of the elongated rod member can be spaced apart such that the ring prosthesis is an open ring. In a further embodiment, the first and second ends of the elongated rod members can be joined together such that the ring prosthesis is a closed ring.

In yet a further example, the elongated rod member can have a substantially cylindrical shape and the substantially flat bioprosthetic tissue can be rolled upon itself to form the substantially cylindrical shape. In yet a further embodiment, the elongated rod member can have a substantially triangular shape and the substantially flat bioprosthetic tissue can be folded upon itself to form the substantially triangular shape. In yet a further embodiment, the elongated rod member can have a substantially rectilinear shape and the substantially flat bioprosthetic tissue can be folded upon itself to form the substantially rectilinear shape. In yet a further embodiment, the tissue can be folded upon itself in an alternating sequence to form the substantially rectilinear shape.

In yet another example, the bioprosthetic tissue can be selected from the group consisting of: pericardium, blood vessels, skin, dura mater, small intestinal submucosa, ligaments, tendons, muscle, ureter, urinary bladder, liver, and heart. In another example, the bioprosthetic tissue can be a pericardium.

In yet another example, the bioprosthetic tissue can be fixed with an aldehyde. In another example, the aldehyde can be a glutaraldehyde. In yet another example, free aldehyde groups in the fixed bioprosthetic tissue can be subjected to a capping treatment with a capping agent. In yet another example, the capping agent can comprise an amine. In yet a further example, the capping treatment can further comprise a reducing agent. In yet a further example, the reducing agent can be a borohydride.

In yet a further example, the bioprosthetic tissue can be plasticized. In a further example, the bioprosthetic tissue can be plasticized with a polyol. In a further example, the polyol can be a glycerol.

The present disclosure also further includes a method for manufacturing a ring prosthesis, which is not covered by the claims. The method can comprise shaping a substantially flat bioprosthetic tissue to form an elongated rod. The bioprosthetic tissue can have a length, a width, a first surface and a second surface opposing the first surface. The rod can comprise a body having first and second ends and a free edge between the first and second ends. The method can further comprise securing the free edge of the rod onto the rod body. The method can further comprise arranging the first and second ends of the rod body in proximity to one another such that the rod is substantially shaped as a ring.

In one example, the shaping can further comprise folding the substantially flat bioprosthetic tissue. In another example, the shaping can comprise rolling the substantially flat bioprosthetic tissue.

In another example, the first surface can be rough or fibrous and the second surface can be smoother than the first surface. In another example, the second surface can be rough or fibrous and the first surface can be smoother than the second surface. In a further embodiment, the first surface forms an exposed surface of the elongated rod and the second surface form an internal surface of the elongated rod. In another embodiment, the second surface can form an exposed surface of the elongated rod and the first surface can form an internal surface of the elongated rod.

In a further example, the step of securing can comprise suturing the free edge of the rod onto the rod body. In yet another example, the step of securing can comprise gluing the free edge of the rod onto the rod body.

In yet another example, the rod can be substantially shaped as an open ring. In yet a further example, the first and second ends of the rod body can be joined together to form a closed ring. In one example, the first and second ends can be joined with sutures. In another example, the first and second ends can be joined with an adhesive.

In yet a further example, a cross-section of the rod body can be substantially circular. In yet another example, a cross-section of the rod body can be substantially triangular. In yet a further example, a cross-section of the rod body can be substantially rectangular.

In yet a further example, the method can further comprise decellularizing the bioprosthetic tissue.

In yet a further example, the bioprosthetic tissue can be selected from the group consisting of: pericardium, blood vessels, skin, dura mater, small intestinal submucosa, ligaments, tendons, muscle, ureter, urinary bladder, liver, and heart. In a further example, the bioprosthetic tissue can be a pericardium.

In yet another example, the bioprosthetic tissue can be fixed with an aldehyde. In another example, the aldehyde is a glutaraldehyde. In another example, free aldehyde groups in the fixed bioprosthetic tissue can be subjected to a capping treatment with a capping agent. In another example, the capping agent can comprise an amine. In another example, the capping treatment can further comprise a reducing agent. In another example, the reducing agent can be a borohydride.

In yet a further example, the bioprosthetic tissue can be plasticized. In a further example, the bioprosthetic tissue can be plasticized with a polyol. In a further example, the polyol can be a glycerol.

All methods disclosed herein which encompass simulations of the methods, for example, for training; testing; demonstration; or device or procedure development, are also not covered by the claims. Methods for treating a patient can include simulating treatment on a simulated human or non-human patient, for example, an anthropomorphic ghost. Examples of suitable simulated patients can include both an entire body, any portion of a body, or at least a portion of an organ, for example, a heart. The simulations can be physical, virtual, or any combination thereof. Examples of physical simulations can include any combination of natural or manufactured whole human or animal cadavers, portions thereof, or cadaver organs. Virtual simulations can include any combination of virtual reality, projections onto a screen or on at least a portion of a physical simulation, or other in silico elements. Some simulations can include non-visual elements, for example, auditory, tactile, or olfactory stimuli.

### BRIEF DESCRIPTION OF THE DRAWINGS

Figure 1A is a top plan view of a mitral annuloplasty ring in accordance with one example.
Figure 1B is a side view of the annuloplasty ring shown in Figure 1A.
Figure 1C is a cross-sectional view of the annuloplasty ring shown in Figure 1A, taken along the lines 1C-1C of Figure 1A.
Figure 1D is a perspective cross-sectional view of an alternative example of the mitral annuloplasty ring shown in Figure 1A.
Figures 1E and 1F are cross-sectional views of alternative examples of the mitral annuloplasty ring shown in Figure 1A.
Figure 2 is a top plan view of a sheet of bioprosthetic tissue in accordance with another example.
Figure 3A is a top plan view of a tricuspid annuloplasty ring in accordance with another example.
Figure 3B is a side view of the annuloplasty ring shown in Figure 3A.
Figure 3C is a cross-sectional view of the annuloplasty ring shown in Figure 3A, taken along the lines 3C-3C of Figure 3A.
Figure 3D is a cross-sectional view of the annuloplasty ring shown in Figure 3A, taken along the lines 3D-3D of Figure 3A.
Figures 3E and 3F are cross-sectional views of alternative examples of the tricuspid annuloplasty ring shown in Figure 3A.
Figure 4A is a partially-exploded perspective view of a sewing ring and a prosthetic heart valve in accordance with another example.
Figure 4B is a top perspective view of the prosthetic heart valve and sewing ring shown in Figure 4A.
Figure 5A is a perspective view a substantially flat biological tissue having opposing surfaces.
Figure 5B is a perspective view of the rolling of the substantially flat biological tissue of Figure 5A.
Figure 5C is a perspective view the rolled, substantially flat biological tissue of Figure 5A.
Figure 5D is a perspective view of the rolled, substantially flat biological tissue of Figure 5C having its free edge sutured.
Figure 5E is a perspective view of the rolled, substantially flat biological tissue of Figure 5D with its free edge sutured and its two ends cut off.
Figure 6 is a perspective view of an annuloplasty ring having a discontinuous or C-shaped periphery in accordance with another example.
Figure 7 is a perspective view of an annuloplasty ring having a continuous periphery in accordance with another example.
Figures 8A is a cross-sectional view of the annuloplasty ring shown in Figures 6 or 7, taken along the lines 8A-8A of Figure 7.
Figures 8B-8C are alternative cross-sectional views of embodiments of the annuloplasty ring shown in Figures 6 or 7.

### DETAILED DESCRIPTION OF SOME EXAMPLES

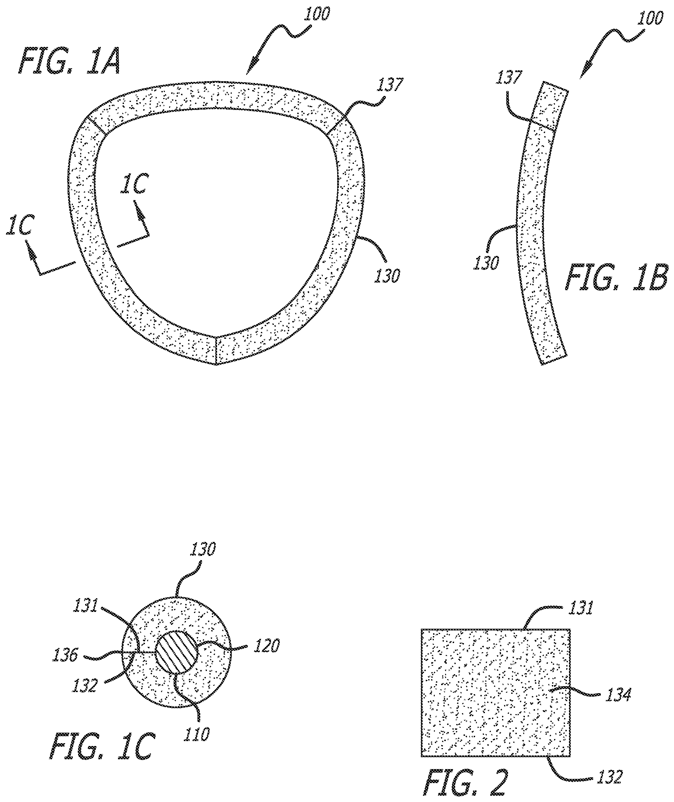

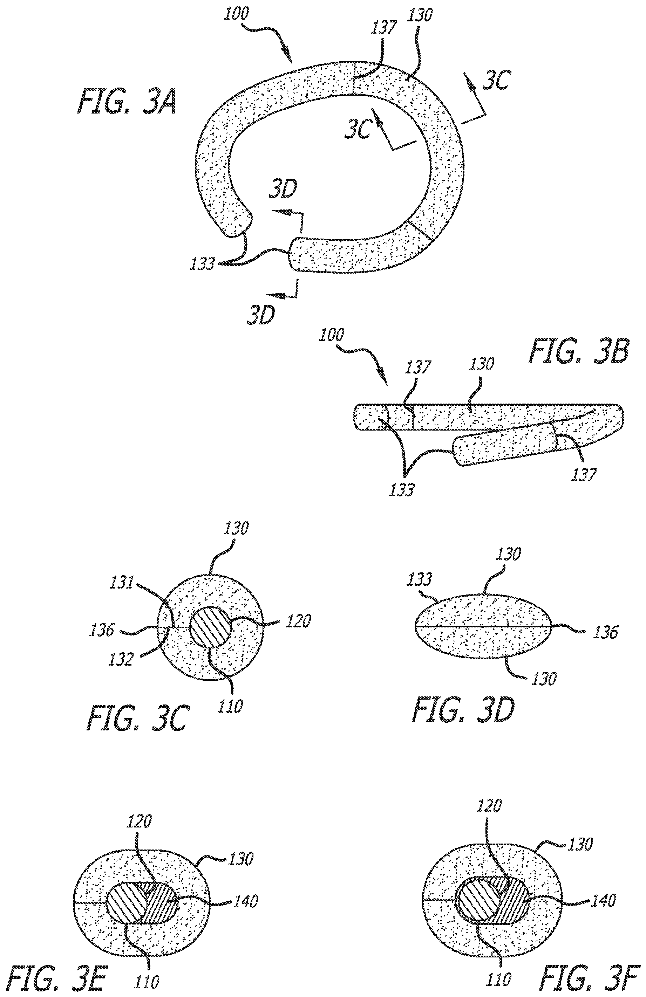

With reference to Figures 1A-1F and 3A-3F of the illustrative drawings, there are shown examples of annuloplasty rings 100. The annuloplasty ring 100 can be suitable for annulus of a valve, such as the mitral annulus (Figures 1A-1F) or the tricuspid annulus (Figures 3A-3F).

Figures 1A-1F illustrate examples of a mitral annuloplasty ring 100 having a continuous or D-shaped periphery. The mitral annuloplasty ring 100 can be shaped to closely mimic the geometry of a healthy mitral annulus, and can be configured to minimize the likelihood of dehiscence while maintaining the shape of a healthy valve annulus.

Figures 3A-3F illustrate examples of a tricuspid annuloplasty ring 100, with a discontinuous or C-shaped periphery, including two free ends 133 that define a gap therebetween. The tricuspid annuloplasty ring 100 can be shaped to closely mimic the geometry of a healthy tricuspid annulus, and can be configured to minimize the likelihood of dehiscence while maintaining the shape of a healthy valve annulus. The tricuspid annuloplasty ring 100 is not complete in about ten percent of the circumference around the anteroseptal commissure of the tricuspid annulus. This is to prevent suture injury to the conduction system. With particular reference to Figure 3B, the tricuspid annuloplasty ring 100 can have a somewhat spiral shape that mimics the shape of a healthy tricuspid annulus.

In one example, the two free ends 133 are separated at a distance to define a gap therebetween. The distance between the two free ends 133 can be about 1%, about 2%, about 3%, about 4%, about 5%, about 6%, about 7%, about 8%, about 9%, about 10%, about 15%, about 20%, about 25%, about 30%, about 35%, about 40%, about 45%, about 50% of a length of the tricuspid annuloplasty ring 100, or in a range that includes and is between any two of the foregoing values.

In one example, the two free ends 133 can be coplanar with the frame 110. In another example, one of the two free ends 133 can be offset from the other one of the free ends 133. The two free ends 133 can be vertically offset from one another at a distance that is about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 35%, about 40%, about 45%, about 50% of a length of the tricuspid annuloplasty ring 100, or in a range that includes and is between any two of the foregoing values.

The annuloplasty ring 100 can comprise a frame 110 having an outer surface 120, and a cover 130 surrounding the frame 110. The cover 130 comprises a bioprosthetic tissue. The bioprosthetic tissue is fixed and non-regenerative. In other examples, not covered by the claims, the bioprosthetic tissue can be regenerative.

The term "regenerative" as it relates to bioprosthetic tissue is understood to mean tissue that permits or even stimulates ingrowth of host cells and tissue into the bioprosthetic tissue after implantation. Thus, "regenerative tissue" can include three-dimensional scaffolds that support the ingrowth of host cells and tissue. In one example, the regenerative tissue can remain after in-growth of host cells and tissue. In another example, the regenerative tissue can partially or completely biodegrade after in-growth of host cells and tissue.

For the fixed and non-regenerative examples, the bioprosthetic tissue can be selected from the group consisting of: pericardium, blood vessels, skin, dura mater, small intestinal submucosa, ligaments, tendons, muscle, ureter, urinary bladder, liver, and heart. For example, the fixed, non-regenerative bioprosthetic tissue can be a pericardium. In one example, the fixed, non-regenerative bioprosthetic tissue can be fixed with an aldehyde such as a glutaraldehyde. In another example, the free aldehyde groups in the fixed, non-regenerative bioprosthetic tissue can be subjected to a capping treatment comprising a capping agent. In a one example, the capping treatment can comprise an amine. In an additional example, the capping treatment can further comprise a reducing agent such as a borohydride. In one example, the fixed, non-regenerative bioprosthetic tissue can be plasticized. In another example, the fixed, non-regenerative bioprosthetic tissue can be plasticized with a polyol such as a glycerol.

In one example, the bioprosthetic tissue can be subjected to a fixation or cross-linking treatment, as a result of which the bioprosthetic tissue is rendered less antigenic and is at least partially or completely cross-linked. The fixation process can also render the tissue non-regenerative. The fixation process is understood to include any chemical, heat or other processes, as a result of which the bioprosthetic tissue is preserved and rendered mechanically and dimensionally stable.

The fixation process can include contacting the tissue with one or more fixatives. Known fixatives include aldehydes, polyaldehydes, diisocyanates, carbodiimides, photo-oxidation agents, and polyepoxide compounds. In a preferred example, the fixative used is glutaraldehyde. Glutaraldehyde-fixed tissue, however, is particularly vulnerable to calcification since glutaraldehyde fixation results in the generation of residual aldehyde groups and labile Schiff bases. The residual aldehydes and Schiff bases can be potential binding sites for calcium. The aldehyde groups can oxidize to carboxylic acid groups, which are known to attract and bind calcium.

Various techniques have therefore been developed to reduce the aldehyde and acid levels of glutaraldehyde-fixed tissues, and thus reduce its propensity to calcify after implantation in the patient.

The fixation process can include adjusting the pH of the glutaraldehyde fixative in solution to reduce the generation of calcium binding sites, as disclosed in U.S. Patent No. 6,878,168 to Edwards Lifesciences. In a preferred example, the pH of the glutaraldehyde fixative in solution is about or provided in a range including and between any two of the following pH values: 4.5, 5, 5.5, 6, 6.5, 7, 7.5, 8, 8.5, and 9.

The fixation process can also further include the addition of a heat-treating step after contacting with the one or more fixatives. Glutaraldehyde-fixed tissue have demonstrated a reduced aldehyde and carboxylic acid content after heat treatment, and thus a marked reduction in calcification after implantation, as compared to glutaraldehyde-fixed tissue without heat treatment. The glutaraldehyde fixative in solution can be heat treated before, during, or after the bioprosthetic tissue is immersed in the solution. The heat treatment can include heating the glutaraldehyde fixative in solution to a temperature provided in a range including and between any two of the following temperatures: 40° C, 41° C, 42° C, 43° C, 44° C, 45° C, 46° C, 47° C, 48° C, 49° C, 50° C, 51° C, 52° C, 53° C, 54° C, 55° C, 56° C, 57° C, 58° C, 59° C, 60° C, 61° C, 62° C, 63° C, 64° C, 65° C, 66° C, 67° C, 68° C, 69° C, 70° C, 71° C, 72° C, 73° C, 74° C, 75° C, 76° C, 77° C, 78° C, 79° C, and 80° C. Exemplary processes for heat treating glutaraldehyde-fixed tissue are described in U.S. Patent No. 6,561,970, issued May 13, 2003 to Edwards Lifesciences. The heat treatment of glutaraldehyde-fixed tissue is also commercially known as the Carpentier-Edwards ThermaFix^{®} (TFX) tissue treatment process from Edwards Lifesciences.

Following or concurrently with the fixation process, the bioprosthetic tissue can be subjected to a capping treatment that comprises a capping agent, a reducing agent, or both. The bioprosthetic tissue can include functional groups that exist either inherently in the bioprosthetic tissue, as a result of being cross-linked or fixed, or as a result of being subjected to any number of chemical or physical processes, including the pre-conditioning, pre-stressing, or pre-damaging processes disclosed in this description. Exemplary processes for treatment with capping and reducing agents are described in U.S. Patent No. 7,972,376.

In one example, the bioprosthetic tissue can be subjected to a capping treatment without the step of fixing or crosslinking the bioprosthetic tissue. In another example, the bioprosthetic tissue can be subjected to the capping treatment before, during, or after the step of fixing or crosslinking the bioprosthetic tissue.

In one example, the capping agent can include any one or a combination of the following: an amine, such as an alkyl amine, amino alcohols and ethanolamine; an amino acid, such lysine and hydroxylysine; an amino sulfonate, such as taurine, amino sulfates, dextran sulfate, and chondroitin sulfate; hydrophilic multifunctional polymers, such as polyvinyl alcohols and polyethyleneimines; a hydrophobic multifunctional polymer; α-dicarbonyls, including methylglyoxal, 3-deoxyglucosone, and glyoxal; hydrazines, such as adipic hydrazide; disuccinimidyl N,N-carbonate; carbodiimides, such as 1-ethyl-3-[3-dimethylaminopropyl]carbodiimide hydrochloride (EDC), N-cyclohexyl-N'-(2-morpholinoethyl)carbodiimide (CMC), and 1,3-dicyclohexyl carbodiimide (DCC); and 2-chloro-1-methylpyridinium iodide (CMPI).

In another example, the capping agent can be any agent that is reactive with a functional group, wherein the functional group is a free aldehyde or a free carboxylic acid. The capping agent can be an amine, such as an alkyl amine or an amino alcohol. The capping agent can be an ethanolamine.

In a further example, the capping agent can be any agent that is reactive with a functional group, wherein the functional group is an amine, a hydroxyl, or a sulfhydryl group. In accordance with this example, the capping agent can comprise a carbonyl functional group. The carbonyl functional group can be an aldehyde or a carboxylic acid and can be selected from a monoaldehyde, a polyaldehyde, a monocarboxylic acid, a polycarboxylic acid, and the like.

Regardless, certain reactions of the capping agent and functional groups can produce labile Schiff bases and it can be desirable to reduce the Schiff bases and replace them with a more stable amine.

Accordingly, the capping treatment of the bioprosthetic tissue can further include treatment with a reducing agent. The reducing agent can be selected to reduce Schiff bases formed from the reaction of the crosslinking agent and the bioprosthetic tissue, the capping agent and the bioprosthetic tissue, and the capping agent and the crosslinking agent. In one example, the bioprosthetic tissue can be treated with the reducing agent, with or without the fixing or crosslinking the bioprosthetic tissue. In another example, the bioprosthetic tissue can be treated with the reducing agent, with or without the capping agent. In a further example, the bioprosthetic tissue can be treated with the reducing agent, with or without both the fixing or crosslinking and capping the bioprosthetic tissue.

The reducing agent can be any one or a combination of agents that comprise a borohydride. In one example, the reducing agent can be one or a combination selected from the group consisting of sodium borohydride, sodium cyanoborohydride, sodium triacetoxyborohydride, sodium bisulfate in acetylacetone, formic acid in formaldehyde, alkyl borohydride, amino borohydride, lithium aminoborohydrides, and an organoborate hydride salt having the formula XBR₃H, where R is an alkyl group and X is lithium, sodium, or potassium. The lithium aminoborohydride can be a lithium dimethylaminoborohydride, a lithium morpholinoborohydride, and a lithium pyrrolidinoborohydride, to name a few. The organoborate hydride salt reducing agent can be a lithium tri-sec-butyl(hydrido)borate, a sodium tri-sec-butyl(hydrido)borate, a potassium tri-sec-butyl(hydrido)borate, or a lithium aluminum hydride.

The bioprosthetic tissue can be subjected to a capping treatment in which it is treated with a capping agent and a reducing agent in a solution. In one example, the capping agent is selected to react with one or more functional groups associated with the bioprosthetic tissue and the reducing agent is selected to reduce Schiff bases. The Schiff bases can be formed from any one or more of the reaction of the crosslinking agent and the bioprosthetic tissue, the reaction of the capping agent and the bioprosthetic tissue, and the reaction of the capping agent and the crosslinking agent. The capping agent can be an amine or an amino alcohol, such as an ethanolamine; the functional groups can be an aldehyde or a carboxylic acid; the reducing agent can be a borohydride, such as a sodium borohydride; and the crosslinking agent can be an aldehyde-containing agent, such as a glutaraldehyde. The capping treating can be performed sequentially with first the capping agent and then the reducing agent in solution or simultaneously with both the capping and reducing agents present in the solution. In one example, the capping treating can be performed with the capping agent and reducing agent in a solution on an orbital shaker operating at about 80 to about 100 rpm for about 4 hours.

Exemplary methods for treating bioprosthetic tissue with capping and reducing agents are described in U.S. Patent No. 7,972,376, issued July 5, 2011 to Edwards Lifesciences Corporation.

The capping treatment can comprise a capping agent, a reducing agent, or both. The capping treatment can be performed after the fixed bioprosthetic tissue has been subjected to a process of pre-conditioning, pre-stressing, or pre-damaging to generate additional acid binding sites, which can subsequently be capped, as described in U.S. Patent Application Publication No. 2008/0302372, published December 11, 2008, entitled "Methods for Pre-Stressing and Capping Bioprosthetic Tissue" to Edwards Lifesciences. In one example, the bioprosthetic tissue can be subjected to a rapid pulsed fluid flow (in the range of about 4 Hz to about 1,500 Hz), repeated flexion of the bioprosthetic tissue valve, elevated temperature (in the range of about 26° C to about 65° C), an acidic solution (pH in the range of about 4 to about 7), alkaline solution (pH in the range of about 8 to about 10), or any combination of the foregoing for the purpose of generating additional acid binding sites, which can be capped, reduced, or both, in a separate treatment process.

The bioprosthetic tissue can further undergo treatment with anhydrous, non-aqueous, or aqueous solutions to substantially, if not completely, dehydrate the bioprosthetic tissue for dry storage. The bioprosthetic tissue following glycerol treatment can contain residual water or moisture within the tissue interstices but can be packaged for dry storage.

In one example, the bioprosthetic tissue can be treated with an anhydrous, non-aqueous, or aqueous solution that comprises glycerol. In one example, the anhydrous, non-aqueous, or aqueous solution can comprise about 25% by volume, 30% by volume, 35% by volume, 40% by volume, 45% by volume, 50% by volume, 55% by volume, 60% by volume, 65% by volume, 70% by volume, 75% by volume, 80% by volume, 85% by volume, 90% by volume, or 95% by volume glycerol. In another example, the anhydrous, non-aqueous, or aqueous solution comprises an amount of glycerol within and including any two of the foregoing values.

In another example, the anhydrous, non-aqueous, or aqueous glycerol solution can comprise alcohol. In one example, the anhydrous, non-aqueous, or aqueous solution can comprise about 5% by volume, about 10% by volume, about 15% by volume, about 20% by volume, about 25% by volume, about 30% by volume, about 35% by volume, about 40% by volume, about 45% by volume, about 50% by volume, about 55% by volume, about 60% by volume, about 65% by volume, about 70% by volume, or about 75% by volume alcohol. In another example, the anhydrous, non-aqueous, or aqueous solution comprises an amount of alcohol within and including any two of the foregoing values. The alcohol can be any one or a combination of C₁, C₂, C₃, C₄, and C₅ alcohols, such as ethanol, propanol, and butanol.

In one example, the solution is a non-aqueous solution of about 75% by volume glycerol and 25% by volume ethanol. The bioprosthetic tissue is immersed in the solution for a period of time sufficient to permit the solution to permeate the bioprosthetic tissue. The bioprosthetic tissue is then removed from the solution to allow removal of excess solution. Suitable treatment for the bioprosthetic tissues is described in U.S. Patent No. 8,007,992, issued August 30, 2011, to Edwards Lifesciences Corporation.

In another preferred example, an aqueous glycerol solution can be used to at least partially dehydrate the tissue, as described in U.S. Patent No. 6,534,004, issued March 18, 2003, issued to The Cleveland Clinic Foundation.

The bioprosthetic tissue can also be treated by means other than the glycerol treatment process described above to dry or dehydrate the bioprosthetic tissue. The terms "dry" or "dehydrate," as used in this disclosure with reference to the bioprosthetic tissue or the implantable bioprosthetic device, is understood to include residual water or moisture that can be present in the bioprosthetic tissue following glycerol or other treatment to reduce the water content of the bioprosthetic tissue. In one example, the water content of the dried or dehydrated bioprosthetic tissue following glycerol or other treatment is about 25% by weight or less, about 20% by weight or less, about 15% by weight or less, about 10% by weight or less, about 9% by weight or less, about 8% by weight or less, about 7% by weight or less, about 6% by weight or less, about 5% by weight or less, about 4% by weight or less, about 3% by weight or less, about 2% by weight or less, or about 1% by weight or less. These percentages are understood to be based on the combined weight of the bioprosthetic tissue and water content.

For the regenerative examples, not falling within the scope of the claimed subject-matter, the bioprosthetic tissue can be an artificial or biological scaffold or a decellularized biological tissue. For example, the bioprosthetic tissue can be selected from the group consisting of: pericardium, blood vessels, skin, dura mater, small intestinal submucosa, ligaments, tendons, muscle, ureter, urinary bladder, liver, and heart. It is understood that the tissue selected is decellularized using any suitable method.

In one example, not falling within the scope of the claimed subject-matter, the regenerative bioprosthetic tissue can be an artificial scaffold. In another example, the artificial scaffold can be a biodegradable polymer scaffold. A biodegradable polymer can include a polymer in which the bonds of the polymer-chain cleave, primarily by aqueous hydrolysis as a result of contact with blood and other bodily fluids at physiological pH (e.g., around 7 to 7.5). This process results in the fragmentation and eventual decomposition of the polymer *in vivo.* The fragmentation and decomposition process can be catalyzed by enzymes or other endogenous biological compounds. In a further example, the biodegradable polymer scaffold can comprise a polyglycolic acid. In an additional example, the artificial scaffold can further comprise one or more extracellular matrix proteins. For example, the extracellular matrix protein can be one or more proteins selected from the group consisting of: hydroxyproline, vitronectin, fibronectin, collagen I, collagen III, collagen IV, collagen VI, collagen XI, collagen XII, fibrillin I, tenascin, decorin, byglycan, versican, asporin, agrin, and combinations thereof.

In the presently claimed invention, the cover 130 is formed from a sheet 134 of bioprosthetic tissue (Figure 2). The sheet 134 has a first edge 131 and a second edge 132. With reference to Figures 1C and 3C, the sheet 134 covers the outer surface 120 of the frame 110, and the first edge 131 and the second edge 132 are sewn, glued, or otherwise joined together to form a seam 136. In some examples of a tricuspid annuloplasty ring 100 (Figures 3A-3D), the sheet 134 can extend beyond the frame 110 to form an enlarged region of covering 130 at the free ends (Figure 3D).

The glue used to form and shape the cover 130, such as joining the first and second edges 131, 132, is preferably one that is biocompatible and strongly bonds tissue in a wet environment (e.g., flowing blood). In one example, the glue is a hydrophobic light-activated adhesive (HLAA). The HLAA can be formed by combining a poly(glycerol sebacate acrylate) (PGSA) with a photo-initiator, such as 2-hydroxy-1-[4-(2-hydroxyethoxy)phenyl]-2-methyl-l-propanone) (IRGACURE 2959, Sigma-Aldrich) to create the HLAA. The HLAA can be a thick gel that can be applied onto the cover and then cross-linked by ultraviolet light. The resulting bond is preferably water-tight yet flexible and stays intact in the face of high pressure and flowing blood.

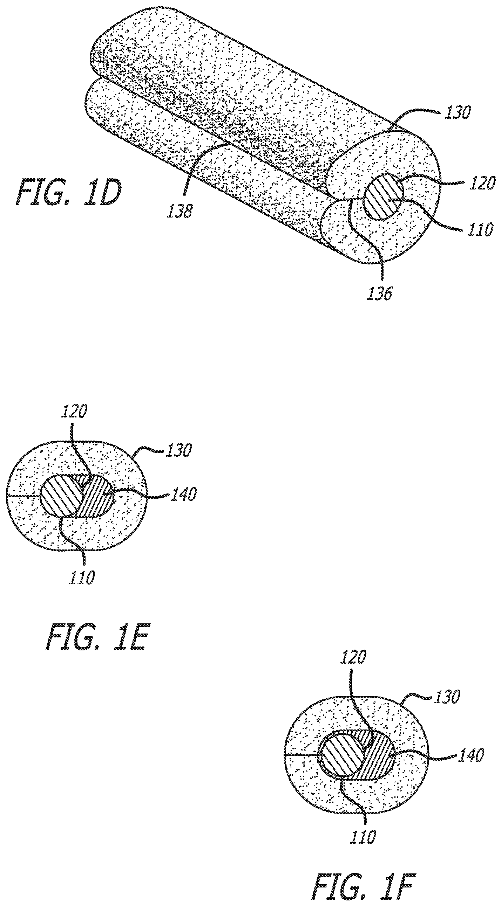

With reference to Figures 1A, 1B, 3A, and 3B, the cover 130 can comprise a plurality of sheets 134 that abut each other at part-interface seams 137. In some examples, the part-interface seams 137 can define markings to aid a surgeon with correct positioning of the ring 100 on the valve anulus. With reference to Figure 1D, in the presently claimed invention, the sheet 134 is dimensioned to permit the first edge 131 and the second edge 132 of the sheet to fold or roll upon each other to form a lip 138. The lip 138 protrudes away from the outer surface 120 of the frame 110.

The outer cover 130 comprising a bioprosthetic tissue can encourage native tissue growth on the annuloplasty ring 100, which can help to maintain the ring 100 in place on the valve annulus. In addition, bioprosthetic-tissue cover 130 can be used in patients with endocarditis or in patients who are otherwise intolerant of cloth-covered implants.

As discussed above, the cover 130 is wrapped around a frame 110. In some examples, the frame 110 can be bioabsorbable. In other examples, the frame 110 can be non- de gradable .

For the non-degradable examples, the frame 110 can comprise one or both of a non-degradable polymer and a non-degradable metal or metal alloy. For example, in one example, the frame 110 can comprise a non-degradable metal or metal alloy selected from the group consisting of: stainless steel, a nickel-based alloy, a cobalt-chromium alloy, a nickel-cobalt-chromium alloy, nitinol, and combinations thereof.

For the bioabsorbable examples, the bioabsorbable frame 110 can comprise a degradable metal or a metal alloy. For example, the degradable metal or metal alloy can comprise one or a combination selected from the group consisting of: magnesium, aluminum, iron, and zinc. The metal or metal alloy can have an ultimate tensile strength of about 30 MPa to about 400 MPa and an elongation of about 0.3 percent to about 170 percent.

In one example, the bioabsorbable frame 110 can be a bioabsorbable material. For example, the bioabsorbable material can be one or a combination of polymers selected from the group consisting of: poly(L-lactide), poly(D-lactide), polyglycolide, poly(L-lactide-co-glycolide), polyhydroxyalkonate, polysaccharides, polyesters, polyhydroxyalkanoates, polyalkelene esters, polyamides, polycaprolactone, polylactide-co-polycaprolactone, polyvinyl esters, polyamide esters, polyvinyl alcohols, modified derivatives of caprolactone polymers, polytrimethylene carbonate, polyacrylates, polyethylene glycol, terminal dials, poly(L-lactide-co-trimethylene carbonate), polyhydroxybutyrate, polyhydroxyvalerate, poly-orthoesters, poly-anhydrides, polyiminocarbonate, and copolymers.

In another example, the bioabsorbable frame 110 can be reinforced with a reinforcing composition. For example, in one example, the reinforcing composition can comprise magnesium or a magnesium alloy.

While the frame 110 is shown to have a circular cross-section in Figures 1C, 1D, and 3C, it should be understood that the frame 110 can have other cross-sectional shapes and thicknesses. For example, in some examples, the frame 110 can have a rounded semi-circular or even polygonal cross-section. In other examples, the shape or thickness of the frame 110 can be uniform across the periphery of the ring 100 or it can vary across the periphery of the ring 100.

In some examples, the ring 100 can further include a suture-permeable interface 140 having one or more layers between the frame 110 and the cover 130. For instance, the suture-permeable interface can comprise an elastomeric sleeve (Figures 1F and 3F) such as a silicone rubber molded around the frame 110. The elastomeric sleeve can provide bulk to the ring for ease of handling and implant, and permit passage of sutures though not significantly adding to the anchoring function of the outer cover 130. In one example, the suture-permeable interface 140 can be provided around at least a portion of the periphery of the ring 100 (Figures 1E and 1F). In another example, the suture-permeable interface 140 can be provided around the entire periphery of the ring 100.

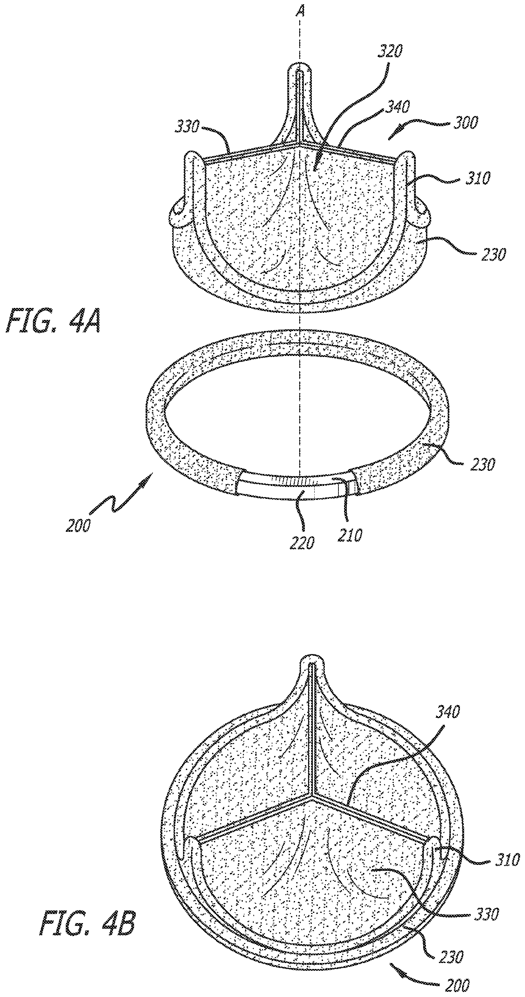

With reference now to Figures 4A and 4B of the illustrative drawings, there is shown a sewing ring 200 for a prosthetic heart valve 300. The prosthetic heart valve 300 can comprise a support frame 310 defining an orifice 320 about an axis A along an inflow-outflow direction. A plurality of leaflets 330 can be mounted for movement on the support frame 310 to provide a one-way valve 340 in the orifice 320. Each of the plurality of leaflets 330 can comprise the bioprosthetic tissue described above.

The sewing ring 200 can be connected to and positioned around the support frame 310 for attaching the heart valve 300 to a valve annulus (not shown). The sewing ring 200 can include a suture-permeable annular member 210 comprising an outer surface 220, and a cover 230 surrounding the annular member 210.

The cover 230 comprises the bioprosthetic tissue described above. For example, as previously outlined, the cover 230 can comprise bioprosthetic tissue that is fixed and non-regenerative, or bioprosthetic tissue that is regenerative. In either case, the cover 230 is wrapped around the annular member 210, which can be molded from a suture-permeable, biocompatible polymer such as silicone. In one example, the annular member 210 can comprise a molded polymer selected from the group consisting of: silicone, polyurethane, and combinations thereof. As with the cover 130 for the annuloplasty ring 100, the cover 230 for the sewing ring 200 can be formed from a sheet 134 of bioprosthetic tissue (Figure 2), as discussed above.

In one example, the cover 230 and the plurality of leaflets 330 are made from the same bioprosthetic tissue.

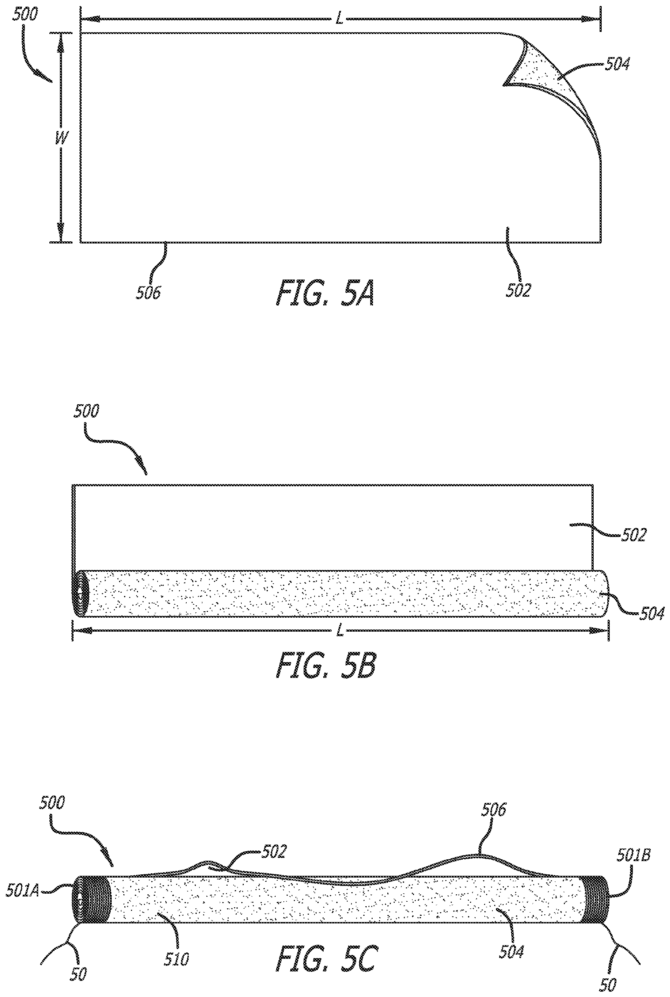

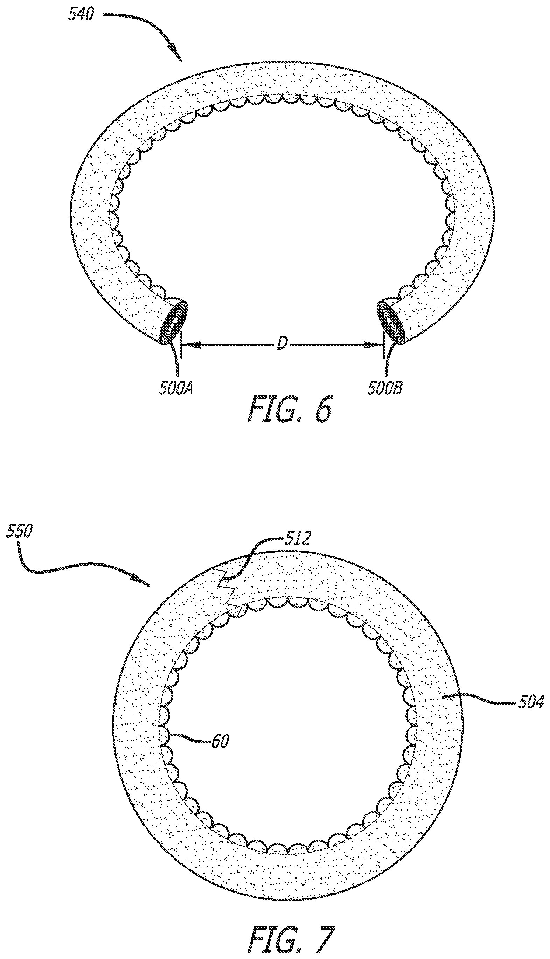

With reference to Figures 5A-5E of the illustrative drawings, there is shown a method of manufacturing various embodiments of ring protheses which includes annuloplasty rings and sewing rings, including those depicted in Figures 6 and 7. As with the annuloplasty rings described herein, ring protheses fabricated in accordance with the depicted method can be suitable for the annulus of a valve, such as the tricuspid annulus (Figure 6) or the mitral annulus (Figure 7). Additionally, ring protheses fabricated in accordance with the depicted manner can be used in fabricating a prosthetic heart valve, as shown in Figures 4A-4B.

Figure 5A depicts an example of a bioprosthetic tissue 500 having a length L and a width W. The bioprosthetic tissue 500 can comprise two opposing surfaces: a first surface 504 and a second surface 502. In one embodiment, the first surface 504 can have a rough texture or can be fibrous and the second surface 502 can be smoother than the first surface 504. Certain biological tissues can be characterized as having such surfaces, such as pericardial tissue and dura mater. These tissues are covered by a cell surface layer only on one side and thus have a smooth and even surface on this side while the opposing side is rough and fibrous. For purposes of cellular integration and infiltration by the host, the rough side can provide a suitable surface.

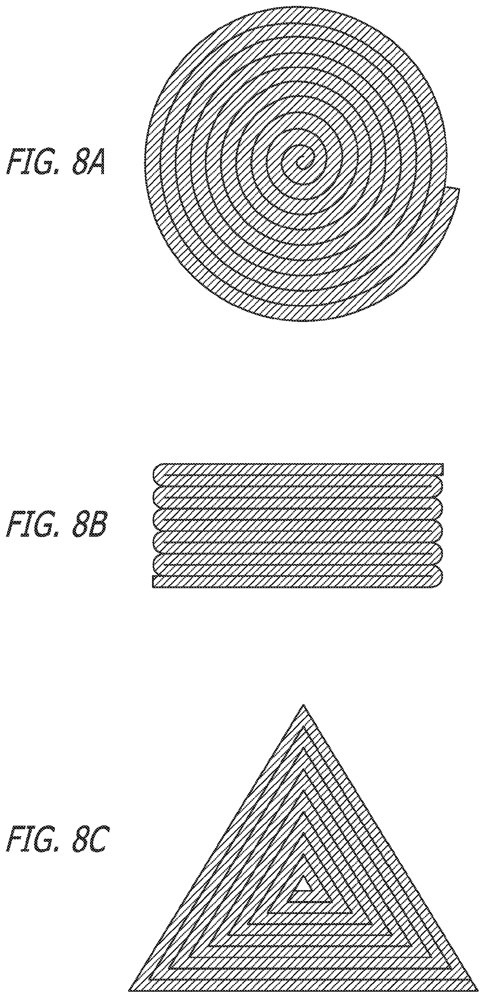

While Figure 5A depicts a perfectly rectilinear shape, it is understood that the biological tissue 500 can be of any shape of a defined length and width so long as it can provide the desired diameter of the resulting ring prosthesis. Figures 5B-5C depict one example of how the biological tissue 500 can be rolled upon itself to produce an elongated rod member 510 having a free edge 506 between the first and second secured ends 501A and 501B. While Figures 5A and 5B depict the elongated rod member 510 as has having a substantially cylindrical cross-sectional shape (Figure 8A), it is understood that the biological tissue 500 can be rolled or folded in a manner to produce other cross-sectional shapes, such as a triangular cross-sectional shape (Figure 8C). Alternatively, the elongated rod member 510 can be formed into a rectilinear cross-sectional shape by alternating folds in an accordion-style (Figure 8B).

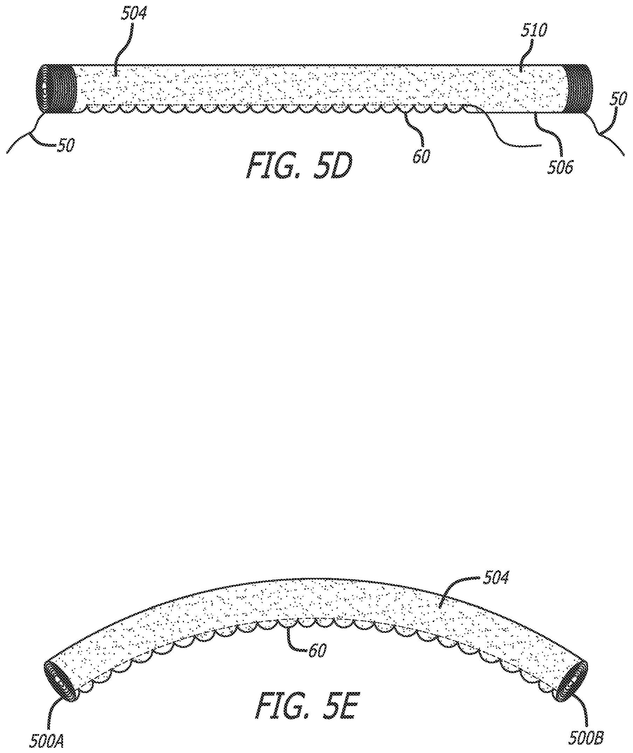

The elongated rod body 510 can be secured at its first and second secured ends 501A, 501B by wrapping with a string or suture 50 so that it may retain its substantially cylindrical shape. Once the first and second ends 501A, 501B are secured, the free edge 506 can be secured to the rod body 510. While Figure 5D depicts the free edge 506 being secured to the rod body 510 with sutures 60, it is understood that the free edge 506 can be secured with any biocompatible substance, such as the adhesives described herein.

As shown in Figures 5E and 6, the free edge 506 may be sutured in such a manner to impart a curvature to the shape of the rod body 510. The two free ends 500A and 500B can be separated at a distance d to define a gap therebetween to produce an open ring 540 as depicted in FIG. 6. The distance D between the two free ends 500A and 500B can be about 1%, about 2%, about 3%, about 4%, about 5%, about 6%, about 7%, about 8%, about 9%, about 10%, about 15%, about 20%, about 25%, about 30%, about 35%, about 40%, about 45%, about 50% of a length of the rod body 510, or in a range that includes and is between any two of the foregoing values. Once the free edge 506 is sutured, the first and second secured ends 501A, 501B can be cut off to produce first and second free ends 500A and 500B. The resulting rod body 510 can consist only of the biological tissue 500 and the sutures 60 which can be made of a dissolvable or bioabsorbable material.

In one example, the two free ends 500A and 500B can be coplanar with the rod body 510. In another example, one of the two free ends 500A, 500B can be offset from the other one of the free ends. The two free ends 500A, 500B can be vertically offset from one another at a distance that is about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 35%, about 40%, about 45%, about 50% of a length of the ring prosthesis, or in a range that includes and is between any two of the foregoing values.

In another example, the two free ends 500A, 500B can be joined together to produce an enclosed ring 550 as depicted in FIG. 7. In one example, the two free ends 500A, 500B can be sutured together to produce a seam 512 therebetween.

It is understood that the bioprosthetic tissue can be treated in any manner as described above either before or after it is formed into a ring prosthesis.

It should be appreciated from the foregoing description that the present disclosure provides improved ring prostheses, including annuloplasty rings that can encourage native tissue growth around the implant, to help maintain the implants in place, and that can be used in patients with endocarditis or in patients who or otherwise intolerant of cloth-covered implants.

Unless defined otherwise, all technical and scientific terms used herein have the same meanings as commonly understood by one of ordinary skill in the art to which this example belongs. The terms "a," "an," and "at least one" encompass one or more of the specified element. That is, if two of a particular element are present, one of these elements is also present and thus "an" element is present. The terms "a plurality of" and "plural" mean two or more of the specified element. The term "or" used between the last two of a list of elements means any one or more of the listed elements. For example, the phrase "A, B, or C" means "A, B, and/or C," which means "A," "B," "C," "A and B," "A and C," "B and C," or "A, B, and C." The term "coupled" generally means physically coupled or linked and does not exclude the presence of intermediate elements between the coupled items absent specific contrary language.

Without further elaboration, it is believed that one skilled in the art, using the proceeding description, can make and use the disclosed subject matter to the fullest extent. The subject matter has been described in detail with reference only to the presently preferred examples. Accordingly, the scope is defined only by the following claims.

## Claims

1. An annuloplasty ring prosthesis comprising:
a frame comprising an outer surface; and
a cover surrounding the frame, the cover comprising a fixed, non-regenerative bioprosthetic tissue wherein:
the cover comprises a sheet having a first edge and a second edge;
the sheet covers the outer surface of the frame;
the first edge and the second edge are joined together to form a seam;
the sheet is dimensioned to permit the first edge and the second edge of the sheet to fold or roll upon each other to form a lip; and
the lip protrudes away from the outer surface of the frame.

2. The annuloplasty ring prosthesis of claim 1, wherein the fixed, non-regenerative bioprosthetic tissue is selected from the group consisting of: pericardium, blood vessels, skin, dura mater, small intestinal submucosa, ligaments, tendons, muscle, ureter, urinary bladder, liver, and heart.

3. The annuloplasty ring prosthesis of claim 2, wherein the fixed, non-regenerative bioprosthetic tissue is a pericardium.

4. The annuloplasty ring prosthesis of any one of the preceding claims, wherein the fixed, non-regenerative bioprosthetic tissue is fixed with an aldehyde.

5. The annuloplasty ring prosthesis of claim 4, wherein the aldehyde is a glutaraldehyde.

6. The annuloplasty ring prosthesis of claim 4, wherein free aldehyde groups in the fixed, non-regenerative bioprosthetic tissue are subjected to a capping treatment comprising a capping agent.

7. The annuloplasty ring prosthesis of claim 6, wherein the capping agent comprises an amine.

8. The annuloplasty ring prosthesis of claim 7, wherein the capping treatment further comprises a reducing agent.

9. The annuloplasty ring prosthesis of claim 8, wherein the reducing agent is a borohydride.

10. The annuloplasty ring prosthesis of any one of the preceding claims, wherein the fixed, non-regenerative bioprosthetic tissue is plasticized.

11. The annuloplasty ring prosthesis of claim 10, wherein the fixed, non-regenerative bioprosthetic tissue is plasticized with a polyol.

12. The annuloplasty ring prosthesis of claim 11, wherein the polyol is a glycerol.

13. The annuloplasty ring prosthesis of any one of the preceding claims, wherein the frame comprises one or both of a non-degradable polymer and a non-degradable metal or metal alloy.

14. The annuloplasty ring prosthesis of claim 13, wherein the frame comprises a non-degradable metal or metal alloy selected from the group consisting of: stainless steel, a nickel-based alloy, a cobalt-chromium alloy, a nickel-cobalt-chromium alloy, Nitinol, and combinations thereof.

## Patentansprüche

1. Anuloplastie-Ringprothese, die Folgendes umfasst:
einen Rahmen, der eine äußere Oberfläche umfasst, und
eine Abdeckung, die den Rahmen umgibt, wobei die Abdeckung ein fixiertes, nicht regeneratives bioprothetisches Gewebe umfasst, wobei:
die Abdeckung ein Blatt mit einer ersten Kante und einer zweiten Kante umfasst;
die Folie die äußere Oberfläche des Rahmens bedeckt;
die erste Kante und die zweite Kante unter Bildung einer Naht miteinander verbunden sind;
das Blatt so dimensioniert ist, dass die erste Kante und die zweite Kante des Blatts unter Bildung einer Lippe aufeinander falten oder rollen können; und
die Lippe von der äußeren Oberfläche des Rahmens wegragt.

2. Anuloplastie-Ringprothese nach Anspruch 1, wobei das fixierte, nicht regenerative bioprothetische Gewebe aus der aus Perikard, Blutgefäßen, Haut, Dura Mater, Dünndarm-Submukosa, Bändern, Sehnen, Muskel, Harnleiter, Harnblase, Leber und Herz bestehenden Gruppe ausgewählt ist.

3. Anuloplastie-Ringprothese nach Anspruch 2, wobei das fixierte, nicht regenerative bioprothetische Gewebe ein Perikard ist.

4. Anuloplastie-Ringprothese nach einem der vorhergehenden Ansprüche, wobei das fixierte, nicht regenerative bioprothetische Gewebe mit einem Aldehyd fixiert ist.

5. Anuloplastie-Ringprothese nach Anspruch 4, wobei der Aldehyd ein Glutaraldehyd ist.

6. Anuloplastie-Ringprothese nach Anspruch 4, wobei freie Aldehydgruppen in dem fixierten, nicht regenerativen bioprothetischen Gewebe einer Verkappungsbehandlung unterzogen werden, die ein Verkappungsmittel umfasst.

7. Anuloplastie-Ringprothese nach Anspruch 6, wobei das Verkappungsmittel ein Amin umfasst.

8. Anuloplastie-Ringprothese nach Anspruch 7, wobei die Verkappungsbehandlung weiterhin ein Reduktionsmittel umfasst.

9. Anuloplastie-Ringprothese nach Anspruch 8, wobei das Reduktionsmittel ein Borhydrid ist.

10. Anuloplastie-Ringprothese nach einem der vorhergehenden Ansprüche, wobei das fixierte, nicht regenerative bioprothetische Gewebe plastifiziert ist.

11. Anuloplastie-Ringprothese nach Anspruch 10, wobei das fixierte, nicht regenerative bioprothetische Gewebe mit einem Polyol plastifiziert ist.

12. Anuloplastie-Ringprothese nach Anspruch 11, wobei das Polyol ein Glycerin ist.

13. Anuloplastie-Ringprothese nach einem der vorhergehenden Ansprüche, wobei der Rahmen ein nicht abbaubares Polymer und/oder ein nicht abbaubares Metall oder eine nicht abbaubare Metalllegierung umfasst.

14. Anuloplastie-Ringprothese nach Anspruch 13, wobei der Rahmen ein nicht abbaubares Metall oder eine nicht abbaubare Metalllegierung ausgewählt aus der aus Edelstahl, einer Legierung auf Nickelbasis, einer Kobalt-Chrom-Legierung, einer Nickel-Kobalt-Chrom-Legierung, Nitinol und Kombinationen davon bestehenden Gruppe umfasst.

## Revendications

1. Prothèse annulaire pour annuloplastie comprenant :
un cadre comprenant une surface extérieure ; et
une couverture entourant le cadre, la couverture comprenant un tissu bioprothétique fixe non régénératif dans laquelle :
la couverture comprend une feuille ayant un premier bord et un second bord ;
la feuille recouvre la surface extérieure du cadre ;
le premier bord et le second bord sont joints ensemble pour former une couture ;
la feuille est dimensionnée pour permettre au premier bord et au second bord de la feuille de se plier ou de rouler l'un sur l'autre pour former une lèvre ; et
la lèvre dépasse de la surface extérieure du cadre.

2. Prothèse annulaire pour annuloplastie selon la revendication 1, dans laquelle le tissu bioprothétique fixe non régénératif est choisi dans le groupe constitué par : péricarde, vaisseaux sanguins, peau, dure-mère, sous-muqueuse de l'intestin grêle, ligaments, tendons, muscles, uretère, vessie, foie et cœur.

3. Prothèse annulaire pour annuloplastie selon la revendication 2, dans laquelle le tissu bioprothétique fixe non régénératif est un péricarde.

4. Prothèse annulaire pour annuloplastie selon l'une quelconque des revendications précédentes, dans laquelle le tissu bioprothétique fixe non régénératif est fixé avec un aldéhyde.

5. Prothèse annulaire pour annuloplastie selon la revendication 4, dans laquelle l'aldéhyde est un glutaraldéhyde.

6. Prothèse annulaire pour annuloplastie selon la revendication 4, dans laquelle des groupes aldéhyde libres dans le tissu bioprothétique fixe non régénératif sont soumis à un traitement de coiffage comprenant un agent de coiffage.

7. Prothèse annulaire pour annuloplastie selon la revendication 6, dans laquelle l'agent de coiffage comprend une amine.

8. Prothèse annulaire pour annuloplastie selon la revendication 7, dans laquelle le traitement de coiffage comprend en outre un agent réducteur.

9. Prothèse annulaire pour annuloplastie selon la revendication 8, dans laquelle l'agent réducteur est un borohydrure.

10. Prothèse annulaire pour annuloplastie selon l'une quelconque des revendications précédentes, dans laquelle le tissu bioprothétique fixe non régénératif est plastifié.

11. Prothèse annulaire pour annuloplastie selon la revendication 10, dans laquelle le tissu bioprothétique fixe non régénératif est plastifié avec un polyol.

12. Prothèse annulaire pour annuloplastie selon la revendication 11, dans laquelle le polyol est un glycérol.

13. Prothèse annulaire pour annuloplastie selon l'une quelconque des revendications précédentes, dans laquelle le cadre comprend un polymère non dégradable et/ou un métal ou un alliage métallique non dégradable.

14. Prothèse annulaire pour annuloplastie selon la revendication 13, dans laquelle le cadre comprend un métal ou un alliage métallique non dégradable choisi dans le groupe constitué par : acier inoxydable, un alliage de base de nickel, un alliage cobalt-chrome, un alliage nickel-cobalt-chrome, Nitinol et leurs combinaisons.
